# EUROPÄISCHE PATENTSCHRIFT

(11) **EP 1 722 694 B1**
(45) Veröffentlichungstag und Bekanntmachung des Hinweises auf die Patenterteilung: **20.05.2009**
(21) Anmeldenummer: 05708654.8
(22) Anmeldetag: 03.03.2005
(51) Int. Cl.: A61B 17/22

(54) **KATHETER ZUM ANSAUGEN, FRAGMENTIEREN UND HINAUSFÖRDERN VON ENTFERNBAREM MATERIAL AUS BLUTGEFÄSSEN**
CATHETER FOR SUCKING, FRAGMENTING REMOVING MATERIAL EXTRACTABLE FROM BLOOD VESSELS
CATHETER CONCU POUR ASPIRER, FRAGMENTER ET EVACUER DE LA MATIERE EXTRACTIBLE DE VAISSEAUX SANGUINS

(30) Priorität: 04.03.2004 CH 369042004; 22.12.2004 CH 217604
(43) Veröffentlichungstag der Anmeldung: 22.11.2006
(60) Teilanmeldung: 08168025.8
(73) Patentinhaber: Straub Medical AG, 7323 Wangs (CH)
(72) Erfinder: STRAUB, Immanuel, CH-7323 Wangs (CH)
(74) Vertreter: Rosenich, Paul
(86) Internationale Anmeldenummer: PCT/IB2005/000543
(87) Internationale Veröffentlichungsnummer: WO 2005/084562

(56) Entgegenhaltungen:
- EP-A- 0 582 533
- WO-A-00/54659
- WO-A-94/24941
- WO-A-96/29941
- US-A- 5 084 052
- US-A- 5 417 703
- US-A1- 2003 114 875

## Beschreibung

Die Erfindung betrifft einen Katheter zum Ansaugen, Fragmentieren und Hinausfördern von entfernbarem Material aus Hohlkörpern, insbesondere Thromben und Embolien aus Blutgefässen, mit einem über einen Führungsdraht unabhängig von diesem axial verschiebbaren, am distalen Ende des Katheters angeordneten Arbeitskopf, welcher wenigstens eine seitliche Öffnung aufweist, wobei der Katheter eine mittels eines vom Arbeitskopf entfernten Drehantriebes einer Antriebseinheit in Rotation versetzbare flexible Förderschraube, sowie mit einem die Förderschraube umgebenden, mit dem Arbeitskopf verbundenen flexiblen Schlauch zur Abfuhr der abgelösten Thromben und Emboliefragmente und einem Schneidwerkzeug aufweist.

Solche Katheter dienen insbesondere zum Behandeln von arteriellen Verschlusserkrankungen durch Ansaugen, Fragmentieren und Hinausfördern von Embolien und Thromben. Sie werden in die Arterie oder Vene eingeführt und vorzugsweise unter Röntgenkontrolle bis zu der verengten oder verschlossenen Stelle, die zu behandeln ist, vorgeschoben. An ihrem vorderen, bzw. distalen Ende ist ein mittels eines Drehantriebes rotierend-antreibbares Fragmentierwerkzeug und ein Arbeitskopf angeordnet.

Bei diesen Kathetern ist in der Regel nach zwei unterschiedlichen Anwendungsbereichen zu unterscheiden:

### A) Atherektomie

Dabei handelt es sich um die Entfernung von in der Regel harten, sich über Jahre an den Gefässwänden festgesetzten Ablagerungen.

### B) Thrombektomie

Dabei handelt es sich um die Entfernung von frischen Blutgerinnseln, die sich an Engpässen stauen und zum Verschluss der Blutgefässe (Embolien) führen.

Ein beispielsweise aus der EP 0 267 539 B1 bekannter Rotationskatheter für die Atherektomie weist als Schneidwerkzeug einen im wesentlichen ellipsenförmigen Schneidfräser auf, dessen Oberfläche mit abrasivem Material versehen ist und der über eine flexible Antriebswelle von einem am proximalen Ende des Katheters angeordneten Drehantrieb mit einer Drehzahl bis zu 160'000/min angetrieben wird. Der Schneidfräser ist mit der flexiblen Antriebswelle verbunden. Die Antriebswelle läuft in einer als Katheterschlauch dienenden schlauchförmigen Hülle. Durch die Antriebswelle hindurch erstreckt sich ein Führungsdraht, der vor dem Einführen des Katheters in ein Blutgefäss eingeführt und bis zur zu behandelnden Stelle oder etwas darüber hinaus vorgeschoben wird und als Führung des Schneidfräsers und der Antriebswelle dient.

Bei diesen bekannten Rotationskatheter ist das Risiko nicht auszuschliessen, dass insbesondere in starken Krümmungen des Blutgefässes die Gefässwand verletzt und unter Umständen sogar perforiert wird.

Ein weiterer aus der US 5 571 122 A bekannter Rotationskatheter weist ein Schneidwerkzeug mit einer Vielzahl von sich in Achsrichtung erstreckenden Schälmessern auf, welches mit einer Drehzahl von ca. 800/min. angetrieben wird. Durch axiales Stauchen des Schneidwerkzeuges können die Schälmesser radial nach aussen ausgebaucht und somit der Aussendurchmesser des Schneidwerkzeuges vergrössert werden. Bei diesem Katheter besteht das Risiko, dass die Schälmesser, insbesondere infolge der relativ langsamen Umfangsgeschwindigkeit rupfen, zerren oder sich mit der Gefässwand verklemmen können, wodurch die Blutgefässe traumatisch reagieren, indem sie sich zusammenziehen und dadurch den weiteren Eingriff verhindern.

Aus der US 5 226 909 ist ein anderer Atherektomie-Katheter bekannt, der an seinem Arbeitskopf ein durch einen Drehantrieb antreibbares und/oder in Achsrichtung verschiebbares, hülsen- oder wendelförmiges Schneidelement aufweist. Mittels eines seitlichen aufblasbaren Ballons wird die Öffnung des Arbeitskopfes gegen die sich an der Gefässwand festgesetzten Ablagerungen gepresst. Diese Ablagerungen werden dann durch Verdrehen oder axiales Vorschieben des Schneidelementes zerkleinert und in einer Kammer gesammelt. Die Kammer muss dann von Zeit zu Zeit durch Zurückziehen des Katheters geleert werden. Eine kontinuierliche Abfuhr von zerkleinertem Ablagerungsmaterial ist nicht vorgesehen.

Die WO 96/29941 A1 zeigt einen Rotationskatheter für die Atherektomie, dessen Arbeitskopf aus einem stillstehenden, mit einem Schlauch verbundenen Stator und einem Rotor besteht. Der Rotor ist gegenüber dem Stator mittels einer hochtourigen Förder/Antriebsschraube verdrehbar. Sowohl der Stator als auch der Rotor weisen an ihrem Umfang in Übereinstimmung bringbare Fenster auf. Durch Scherung zwischen einer Schneide am Rotor und einer Gegenschneide an den Öffnungen des Stators wird eine Zerkleinerung der in die Öffnungen hineinragenden, bzw. eingesaugten Teile bewirkt. Der Rotor kann den Stator aussen umgeben ("Aussenläufer") oder im Inneren des Rotors angeordnet sein ("Innenläufer").

Katheter mit innen- und aussenlaufenden Rotoren mit Schneiden, welche um die Katheterachse arbeiten, haben den Nachteil, dass sie Blut und Verschlussmaterial aufwirbeln, sodass der Blutfluss von proximal zu distal Partikel abschwemmen kann, welche in anderen Bereichen des Blutkreislaufs, besonders in kleinen Blutgefässen, erneut Verstopfungen und Durchblutungsprobleme erzeugen können

Weitere Dokumente zum Stand der Technik sind: EP 0 310 285 A2;
EP 0 448 859 A2, EP 0 669 106 A2, EP 0 680 730 A2, EP 0 669 106 B1,
EP 0 739 603 A1, WO 02/49690 A2, US 4,857,046 und US 5,100,426.

Bisher bekannte Rotationskatheter mit umlaufenden messerartigen Elementen oder Schneidfräsern benötigen einen relativ starken Drehantrieb, was zur Kompensation des Reaktionsmomentes, welches beim Schneiden von Partikeln entsteht, wiederum einen stärkeren verdrehsteiferen Katheterschlauch nötig macht, damit der Katheterschlauch nicht um die Längsachse verdreht wird. Ein stärkerer, bzw. verdrehsteiferer Schlauch ist jedoch zwingend weniger biegeelastisch, wodurch der Katheter u.U. in den Krümmungen von Blutgefässen nachteilig ist.

Es soll eine Bauteilreduktion stattfinden und die Verletzungsgefahr von Blutgefässwänden minimiert werden. Die Vorteile von bekannten Systemen, insbesondere jene des Systems nach WO 96/29941 A1 sollen jedoch beibehalten bleiben. Dokument US-A-5417703 offenbart einen Katheter gemäß dem oberbegriff des ersten Anspruchs. Die Förderschraube im Bereich des Arbeitskopfes ist als mit der Öffnung des Arbeitskopfes zusammenwirkendes scherendes Schneidwerkzeug ausgebildet, um zwischen den peripheren Rändern der Förderschraube und Rändern der Öffnungen die eindringenden Materialien, bzw. angesaugten und/oder abgelösten Thromben und Embolien kontinuierlich zu zerkleinern. - Die Förderwirkung der Förderschraube bleibt wie beim Aufbau gemäss der WO 96/ 29941 A1 erhalten.

Die Förderschraube übernimmt somit eine Zusatzfunktion, nämlich die Zerkleinerung der durch die Wirkung des Unterdruckes in Förderrichtung in die Öffnungen eingesaugten Ablagerungs- und Gewebeteile. Im Unterschied zum Stand der Technik erfolgt diese Zerkleinerung nicht rhythmisch beim Aufeinandertreffen von Schneiden, sondern kontinuierlich. Die innendrehende und innenschneidende Förderschraube im Arbeitskopf saugt an und zerkleinert die Ablagerungen, ohne dass es ausserhalb zu Verwirbelungen kommt. Die Gefahr, dass Partikel abgeschwemmt werden und es distal zu Durchblutungsstörungen infolge der Katheterintervention kommt, fällt somit weg.

Somit treten praktisch auch keine Schwingungen auf, welche man bei Blutgefässen grundsätzlich vermeiden möchte. Da durch das kontinuierliche Schneiden ein geringeres Reaktionsdrehmoment nötig ist, kann der Schlauch auch dünnwandiger und elastischer sein. Dies insbesondere auch deshalb, weil die beim Fragmentieren entstehende Schnittkraft hauptsächlich in axialer Richtung (proximal) und nicht wie beim beschriebenen stand der Technik, in Umfangsrichtung wirkt. Die Torsionsbelastung des Schlauches ist somit sehr gering.

Gegenüber dem aus der WO 96/29941 A1 bekannten Stand der Technik wird wenigstens eines der Bauteile des Arbeitskopfes, nämlich der Rotor eingespart. Dies führt zu einer Vereinfachung und Kosteneinsparung. Auch kann es mangels Rotor nicht zu einem Verklemmen zwischen Rotor und Stator kommen. Ausserdem ermöglicht die erfindungsgemässe Konstruktion eine Reduktion des Aussendurchmessers auf bisher nicht realisierbare kleine Dimensionen. Solch kleine Dimensionen sind beispielsweise für die Behandlung von Herzkranzgefässen erforderlich

Die Förderschraube ist zweckmässig im Bereich des Arbeitskopfes als mit der Öffnung des Arbeitskopfes zusammenwirkendes, scherendes Schneidwerkzeug ausgebildet, das im Betriebszustand zwischen den peripheren Rändern der Förderschraube und Rändern der Öffnungen die eindringenden Materialien, bzw. angesaugten und/oder abgelösten Thromben und Embolien kontinuierlich zerkleinert und entlang der Förderfläche in Richtung des proximalen Endes abführt.

Der Erfindung liegt die Aufgabe zugrunde, einen Katheter der Eingangs genannten Art, insbesondere für die Thrombektomie, zu schaffen, der keine aussenumlaufen de Schneidmesser, Schneidfräser oder dgl. hat, atraumatisch arbeitet und Thromben sowie Embolien in den Blutgefässe ansaugen, fragmentieren und durch den Katheterschlauch kontinuierlich aus dem Gefäss fördern kann.

Die Förderschraube ist erfindungs gemäß im Bereich des Arbeitskopfes als mit der Öffnung des Arbeitskopfes zusammenwirkendes, scherendes Schneidwerkzeug ausgebildet, das im Betriebszustand zwischen den peripheren Rändern der Förderschraube und Rändern der Öffnungen die eindringenden Materialien, bzw. angesaugten und/oder abgelösten Thromben und Embolien kontinuierlich zerkleinert und entlang der Förderfläche abführt und dass die seitliche Öffnung des Arbeitskopfes als L-förmiger Schlitz mit einem sich im wesentlichen in Längsrichtung und einem sich entlang eines Teils des Umfanges erstreckenden Schenkel ausgebildet ist. Die zu entfernenden Thromben und Embolien könne somit entlang des sich in Längsrichtung erstreckenden Schenkels ins Innere des Arbeitskopfes hineingezogen, bzw. eingesaugt und dann mittels der Förderschraube an der proximalen Kante des sich in Umfangsrichtung erstreckenden Schenkels abgeschert werden.

Das Verhältnis der Breite des sich in Längsrichtung erstreckenden Schenkels zur Breite des sich in Umfangsrichtung erstreckenden Schenkels zwischen 1,0 und 1,3 beträgt. Somit ein guter Transport der eingesaugten Thromben und Embolien in Richtung des proximalen Endes und anschliessend ein sauberes Abscheren ermöglicht.

Der distale Teil der Förderschraube ist im Bereich des Arbeitskopfes ist im Aussendurchmesser gegenüber dem Innendurchmesser des vorzugsweise zylindrischen Arbeitskopfes passgenau ausgebildet, so dass der Aussendurchmesser der Förderschraube zur Innenmantelfläche des Arbeitskopfes ein nur minimales Durchmesserspiel ergibt. Dadurch wird verhindert, dass sich fragmentierte Elemente zwischen der Förderschraube und dem Innendurchmesser des Arbeitskopfes verklemmen können.

Die Kanten an der Aussenseite der Förderschraube sind im Bereich der Öffnung des Arbeitskopfes vorteilhafterweise scharfkantig ausgebildet. Dies ermöglich ein gutes und sauberes Abscheren zu entfernenden, meisten sehr zähen Thromben und Embolien

Der Arbeitskopf ist zweckmässigerweise gegen sein distales Ende verjüngt. Dadurch wird erreicht, dass der Katheter auch in engen Krümmungsradien der Gefässe ohne grösseren Widerstand gleitend vorgeschoben werden kann. Dadurch kann er sich an der Gefässwandung, bzw. an Vorsprüngen, nicht einhaken.

Die Kanten der seitlichen Öffnungen sind auf der Innenseite vorteilhaft wenigstens bereichsweise scharfkantig ausgebildet. Dadurch wird zusammen mit der Peripherie der Förderschraube ein sauberer Schervorgang zum Fragmentieren der Thromben oder Embolien ermöglicht. Die Öffnungen im Katheterkopf sind so konstruiert, dass die mit hoher Drehzahl rotierende Förderschraube Thromben und Embolien, welche angesaugt werden, an den inneren scharfen Kanten der Öffnungen und dem Aussendurchmesser der Förderschraube in Stücke fragmentiert werden. Diese Stücke werden durch den herrschenden Sog und die Schraubenförderung in Richtung des Drehantriebes gefördert.

Die Kanten der seitlichen Öffnung sind auf der Mantelseite des Arbeitskopfes zweckmässigerweise wenigstens bereichsweise verrundet. Dies ermöglicht eine wirbelfreie Strömung der zu entfernenden Ablagerungen sowie anderer Körperflüssigkeiten im Bereich des Arbeitskopfes.

Es ist zweckmässig, dass der Schlitz wenigstens teilweise in axialer Richtung des Arbeitskopfes verläuft. Durch Verändern von Länge und Breite kann der Schlitz entsprechend den unterschiedlichen Anforderungen der verschiedenen Anwendungen angepasst werden.

Eine vorteilhafte Ausführung besteht darin, dass der Schlitz relativ zur Längsachse des Arbeitskopfes wenigstens zum Teil entlang einer Schraubenlinie ausgebildet ist. Durch den Steigungswinkel, bzw. den Drehsinn der Schraubenlinie, ist ebenfalls eine optimale Anpassung an die jeweiligen Gegebenheiten möglich. Der Drehsinn der Schraubenlinie kann gleich oder ungleich gerichtet sein wie der Drehsinn der Förderschraube. Ein gleicher Drehsinn ergibt einen ziehenden Schnitt über eine grössere Schneidlänge. Dies ist insbesondere für zähes oder faseriges abzutragendes Material von Vorteil. Ein entgegengesetzter Drehsinn ergibt einen kurzen Schnitt und ist eher für sprödes Material geeignet.

Für bestimmte Anwendungen ist es zweckmässig, dass die Breite des Schlitzes zum proximalen Ende des Arbeitskopfes hin abnimmt. Die in den Schlitz eingesaugten Ablagerungen wie Thromben oder Embolien werden somit zum proximalen Ende hin gegen einen Engpass gedrängt, der eine verbesserte Fragmentierung der Ablagerungen ermöglicht.

Gemäß der Erfindung ist der Schiltz L-förmig ausgebildet . Der Schlitz kann beispielsweise aus einem in Achsrichtung verlaufenden Teil und einem mit diesem verbundenen, in Umfangsrichtung verlaufenden Teil bestehen.

Am distalen Endbereich des Arbeitskopfes ist zweckmässigerweise wenigstens eine vom distalen Ende ausgehende, in die seitliche Öffnung mündende nutförmige Ausnehmung ausgebildet. Diese nutförmige Ausnehmung bildet einen Kanal, durch den Thromben, Embolien und/oder andere Ablagerungen somit auch vom distalen Ende her angesaugt, zum Bereich der seitlichen Öffnung gelangen und durch das Zusammenwirken der Förderschraube mit dem Arbeitskopf zerkleinert werden können.

Es ist vorteilhaft, wenn die Tiefe der nutförmigen Ausnehmung zum proximalen Ende des Arbeitskopfes hin zunimmt. Dies kann einerseits dadurch erfolgen, das sich der Arbeitskopf gegen das distale Ende hin verjüngt, oder dass die Grundfläche der Ausnehmung gegenüber der Längsachse des Arbeitskopfes geneigt angeordnet ist. Durch die zunehmende Tiefe wird der Durchflussquerschnitt zum proximalen Ende hin vergrössert und somit der Abtransport der Ablagerungen erleichtert.

Die Breite der nutförmigen Ausnehmung ist zweckmässigerweise grösser als die Sehne des Innendurchmessers des Arbeitskopfes im Bereich des Nutbodens. Dadurch entstehen saubere Kanten, denen entlang die Ablagerungen ins Innere des Arbeitskopfes gesaugt und um dann dort fragmentiert zu werden.

Der Arbeitskopf ist vorteilhaft mit dem Schlauch axial zug- und druckfest verbunden. Da auf den Schlauch im Gegensatz zum Stand der Technik praktisch nur ein geringes Reaktions-Drehmoment ausgeübt wird, sind die Anforderungen an die Verbindung zwischen dem Arbeitskopf und dem Schlauch sowie an die Drehsteifigkeit des Schlauches selbst relativ gering, sodass beispielsweise eine einfache Press- oder Klebeverbindung möglich ist und der Schlauch sehr elastisch sein kann.

Im Schlauch entsteht infolge der durch die Förderschraube bewirkten Strömung ein Unterdruck. Um die Flexibilität des Schlauches zu steigern, ist es zweckmässig, dass der Schlauch wenigstens abschnittsweise eine Armierung aufweist. Durch die Armierung kann ausserdem die Wandstärke des Schlauches dünner gehalten und die Flexibilität auch dadurch erhöht werden. Eine Armierung wirkt ausserdem stabilisierend auf das Spiel zwischen der Innenwand des Schlauches dem Aussendurchmesser der Förderschraube.

Die Armierung ist vorteilhaft als metallische Wendel ausgebildet. Eine solche Wendel weist eine hohe Flexibilität in Biegerichtung sowie eine gute Zug- und Druckfestigkeit auf.

Für die Herstellung sowie auch für den Gebrauch beim Einführen des Katheters ist es zweckmässig, die Armierung auf der Innenseite des Schlauches anzuordnen. Dadurch wird eine glatte Oberfläche an der Aussenseite des Katheters ermöglicht. Die Armierung kann jedoch auch vollständig in Kunststoff eingebettet sein.

Eine vorteilhafte Ausführung besteht darin, dass der Schlauch zweiteilig ausgebildet ist, wobei der proximale Teil als reiner Kunststoffschlauch und der distale, dem Arbeitskopf zugewandte Teil als metallische Federwendel mit dünnwandiger elastischer Kunststoff-Ummantelung ausgebildet ist. Somit ist der distale Teil des Schlauches besonders biegsam und der Katheter kann praktisch mühelos auch um enge Kurven vor- und zurückgeschoben werden.

Der Arbeitskopf und/oder die Förderschraube bestehen zweckmässigerweise aus Metall. Dabei sind insbesondere rostfreie Stähle oder andere korrosionsbeständige Legierungen geeignet.

Der Arbeitskopf kann alternativ hinsichtlich verbesserten Werkstoffeigenschaften auch aus gesinterter Keramik oder Metallkeramik aufgebaut sein oder eine hochfeste Verschleiss-Schutzschicht aufweisen.

Weitere Ausbildungen der Erfindung und Varianten dazu sind in den abhängigen Patentansprüchen sowie in den Figuren und der Zeichnungsbeschreibung angegeben.

Im obigen Text wird zwar auf einen Katheter zum Ansaugen, Fragmentieren und Hinausbefördern, insbesondere aus menschlichen Blutgefässen, Bezug genommen; die Erfindung ist jedoch nicht darauf eingeschränkt, sondern steht vielmehr auch anderen Benutzern für analoge Anwendungen im medizinischen Sektor (z.B. Wiederöffnen verstopfter Organbereiche, wie bspw. Harn-, Gallen- oder Eileiter sowie Gefässprothesen und sog. "Stents") offen. Die Patentansprüche sind dementsprechend breit auszulegen.

Die Bezugszeichenliste und die Zeichnung bilden zusammen mit den in den Ansprüchen beschriebenen, beziehungsweise geschützten Gegenständen integrierende Bestandteile der Offenbarung dieser Anmeldung.

### Figurenbeschreibung

Die Figuren werden zusammenhängend und übergreifend beschrieben. Gleiche Bezugszeichen bedeuten gleiche Bauteile, Bezugszeichen mit unterschiedlichen Indices geben funktionengleiche Bauteile an.

### Es zeigen dabei beispielhaft:

- Fig.1: den symbolischen Gesamtaufbau eines Gerätes mit einem nicht erfindungsgemässen Katheter
- Fig.2: den Arbeitskopf eines Katheters gemäss Fig. 1, in Ansicht;
- Fig.3: den Arbeitskopf gemäss Fig. 2, im Längsschnitt;
- Fig. 4 und 5:: eine Variante des Arbeitskopfes gemäß der Erfindung, in perspektivischer Ansicht;
- Fig. 6 bis 10:: eine Variante des Arbeitskopfes mit rechteckiger seitlicher Öffnung nicht gemäß der Erfindung;
- Fig. 11 bis 15:: eine Variante des Arbeitskopfes gemäss Fig. 6. bis 10 nicht gemäß der Erfindung, mit schmalem, sich in Längsrichtung erstreckendem Schlitz;
- Fig. 16 bis 20:: eine Variante des Arbeitskopfes mit etwa quadratischer seitlicher Öffnung;
- Fig. 21 bis 25:: eine Variante des Arbeitskopfes mit schlitzförmiger, sich in Umfangsrichtung erstreckender Öffnung nicht gemäß der Erfindung;
- Fig. 26 bis 30:: eine Variante des Arbeitskopfes mit einer nutförmigen, vom distalen Ende ausgehenden in die seitliche Öffnung mündenden Ausnehmung, nicht gemäß der Erfindung;
- Fig. 31 bis 35:: eine Variante des Arbeitskopfes mit einer als Längsschlitz ausgebildeten seitlichen Öffnung und einer nutförmigen, vom distalen Ende ausgehenden in die seitliche Öffnung mündenden Ausnehmungs, nicht gemäß der Erfindung ;
- Fig. 36 bis 40:: eine Variante des Arbeitskopfes mit einer etwa dreieckförmigen seitlichen Öffnung mündenden Ausnehmung, deren Breite sich zum proximalen Ende hin verjüngt, nicht gemäß der Erfindung;
- Fig. 41 bis 45:: eine Variante des Arbeitskopfes mit einer seitlichen Öffnung, die aus einem sich in Achsrichtung erstreckenden und einem sich über einen Teil des Umfanges erstreckenden Bereich besteht, gemäß der Erfindung;
- Fig. 46 bis 50:: eine Variante des Arbeitskopfes analog Fig. 41 bis 45, wobei der sich über einen Teil des Umfanges erstreckende Bereich in der Gegenrichtung; gemäß der Erfindung;
- Fig. 51 bis 55:: eine Variante des Arbeitskopfes analog Fig. 41 bis 45, wobei der sich in Längsrichtung erstreckende Bereich wesentlich länger ist, gemäß der Erfindung;
- Fig. 56 bis 60:: eine Variante des Arbeitskopfs analog Fig. 51 bis 55, wobei der sich über einen Teil des Umfanges erstreckende Bereich in der Gegenrichtung gemäss dem Aufbau gemäss Fig. 51 bis 55 verläuft;
- Fig. 61 bis 65:: eine Variante des Arbeitskopfes mit einer sich entlang einer Schraubenlinie erstreckenden seitlichen Öffnung nicht gemäß der Erfindung;
- Fig. 66 bis 70:: eine Variante des Arbeitskopfes analog Fig. 61 bis 65, wobei der entlang einer Schraubenlinie verlaufende Bereich der Öffnung am distalen Ende in einen sich in Achsrichtung verlaufenden Bereich mündet gemäß der Erfindung;
- Fig. 71 bis 75:: eine Variante des Arbeitskopfes analog Fig. 66 bis 70, wobei der entlang einer Schraubenlinie verlaufende Bereich der Öffnung im entgegengesetzten Drehsinn gereichtet ist und
- Fig. 76 bis 80:: eine Variante des Arbeitskopfes analog Fig. 66 bis 70, wobei eine vom distalen Ende ausgehende, nutförmige Ausnehmung in die entlang einer Schraubenlinie verlaufende Öffnung mündet.

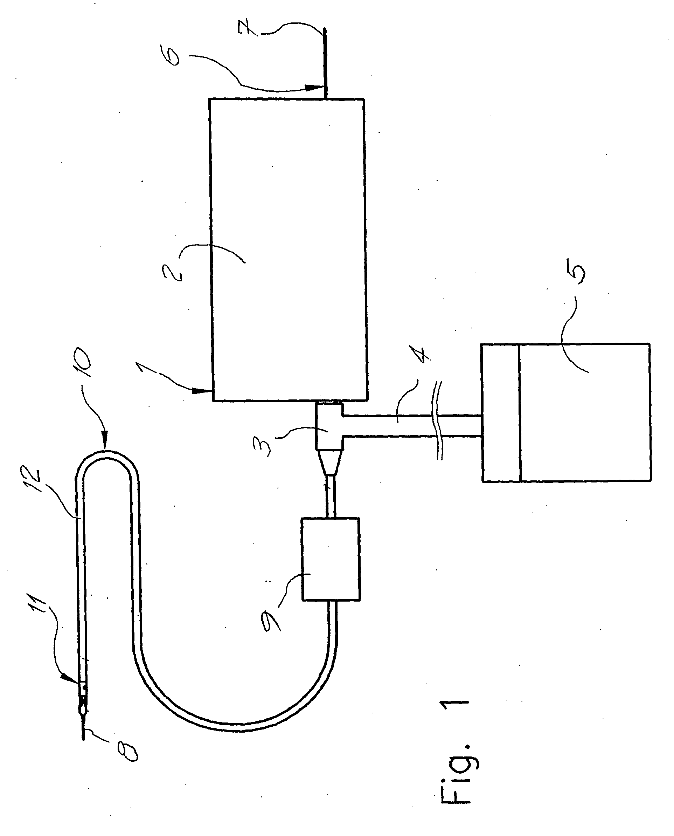

Die Fig.1 zeigt schematisch den Gesamtaufbau eines medizinischen Gerätes zur Verwendung der erfindungsgemässen Katheter. Das Gerät weist eine Antriebseinheit 1 mit einem Drehantrieb 2 auf. Am vorderen Ende des Drehantriebs befindet sich eine Spritzkammer 3. Diese ist über einen Abfuhrkanal 4 mit einem Sammelbehälter 5 verbunden. Ein die Antriebseinheit durchdringender Führungsdraht 6 weist ein proximales (hinteres) Ende 7 und ein distales (vorderes) Ende 8 auf. Der Spritzkammer 3 ist eine bewegliche Einführschleuse 9 vorgelagert. - Dieser Aufbau entspricht im wesentlichen jenem der WO 96/29941 A1. Von dort können auch konstruktive Details übernommen werden.

Ein insgesamt mit 10 bezeichneter Katheter besteht im wesentlichen aus einem flexiblen Schlauch 12 und einem damit zug- und druckfest verbundenen Arbeitskopf 11. Der Führungsdraht 6 durchsetzt den Katheter 10, wobei das distale Ende 8 den Arbeitskopf 11 überragt.

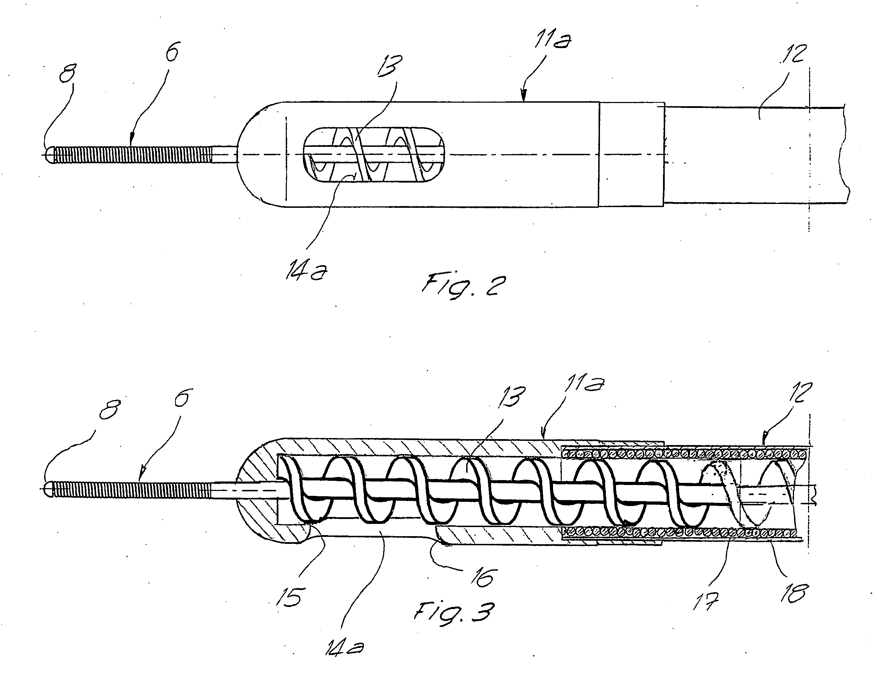

Der aus Fig. 2 und 3 ersichtliche, vergrössert und teilweise im Schnitt dargestellte Arbeitskopf 11 a weist eine seitliche Öffnung 14a auf. Eine wendelförmig ausgebildete Förderschraube 13 umgibt den Führungsdraht 6 und ist im Aussendurchmesser genau auf den Innendurchmesser des Arbeitskopfes 11a abgestimmt. Die Öffnung 14a weist eine innere Kante 15 auf, die scharfkantig ausgebildet ist und einen äusseren Rand 16, der gerundet ist. An der Kante 15 werden die Ablagerungen, die durch den von der Fördereschraube13 erzeugten Unterdruck ins Innere des Arbeitskopfes 11 a gesaugt werden, durch die mit der Kante 15 zusammenwirkende Peripherie der Förderschraube 13 durch Abscheren fragmentiert und mittels der Förderschraube 13 in Richtung der Antriebeinheit 1 durch den Schlauch 12 befördert.

Der aus Fig. 3 ersichtliche Schnitt zeigt den Aufbau des Schlauches 12. Dieser besteht vorzugsweise aus einer beispielsweise aus einem feinen Draht gewickelten Armierung 17 und einer dünnen Ummantelung 18 aus Kunststoff. Dieser Aufbau ergibt eine sehr hohe Flexibilität des Schlauches 12, welche insbesondere im distalen Bereich des Katheters 10 von Vorteil ist. Der proximale Bereich des Schlauches kann aus Herstellungs- und Kostengründen auch als gewöhnlicher dickerer Kunststoffschlauch ausgebildet sein, wobei die beiden Bereiche beispielsweise durch Schrumpfen oder Kleben miteinander verbunden werden können. Eine Variante ist das gemeinsame Überziehen des Armierungsschlauches und des anschliessenden proximalen Teils des Schlauches mit einem dünnen, eng anliegenden Überzug.

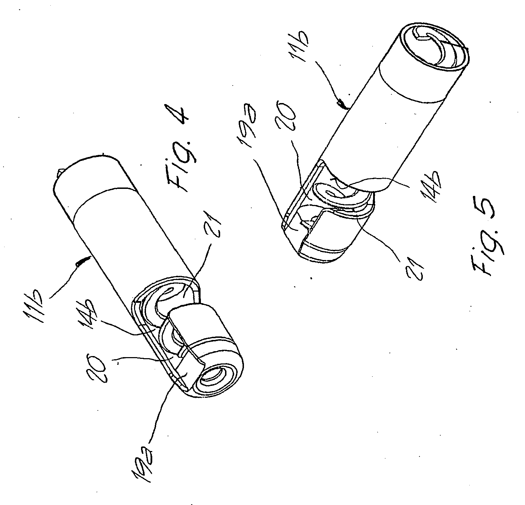

Der aus Fig. 4 und 5 ersichtliche Arbeitskopf 11 b weist eine Öffnung 14b auf, welche im wesentlichen aus einem Längsschlitz 20 und einem sich entlang einem teil des Umfanges erstreckenden Umfangsschlitz 21 besteht. Eine vom distalen Ende ausgehende nutförmige Ausnehmung 19a mündet in den Längsschlitz 20. Diese Ausbildung ermöglicht eine Erfassung der dem Arbeitskopf 11 b vorgelagerten Ablagerungen. Der Arbeitskopf 11 b verjüngt sich gegen das distale Ende. Dies erleichtert das Vorschieben des Katheters in dem freizumachen Leiter, bzw. Blutgefäss.

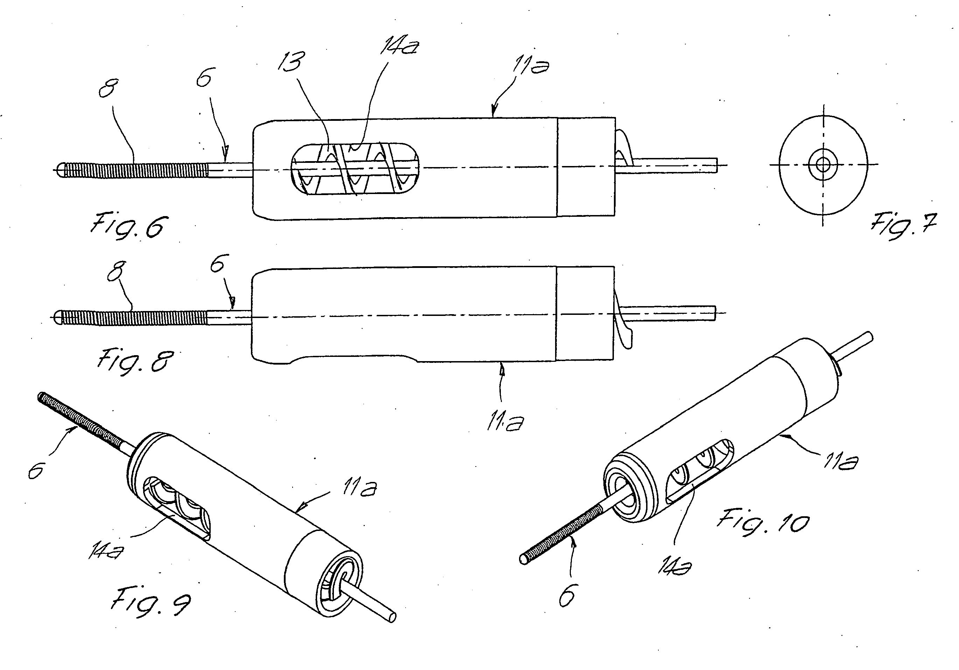

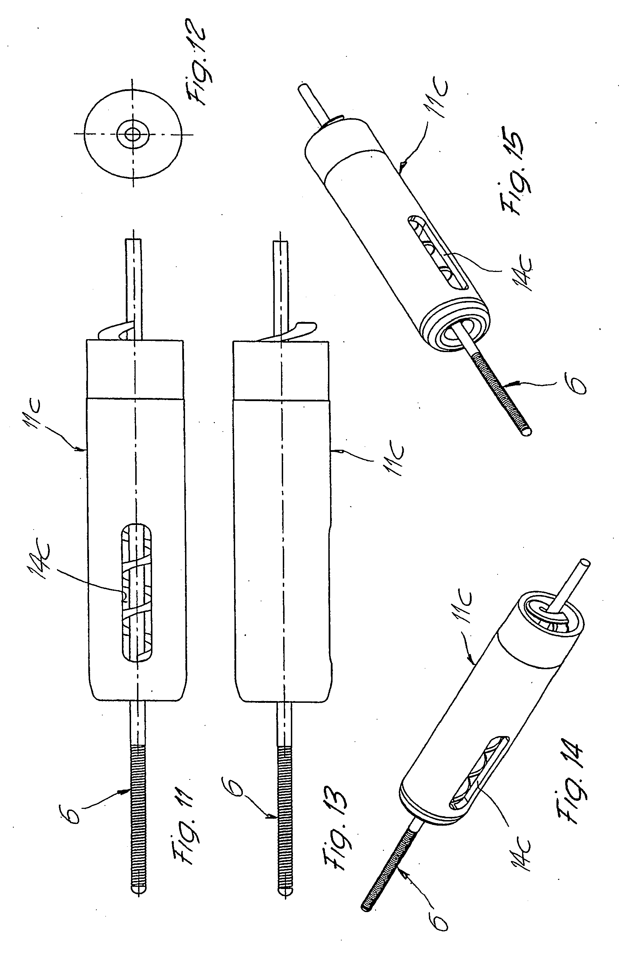

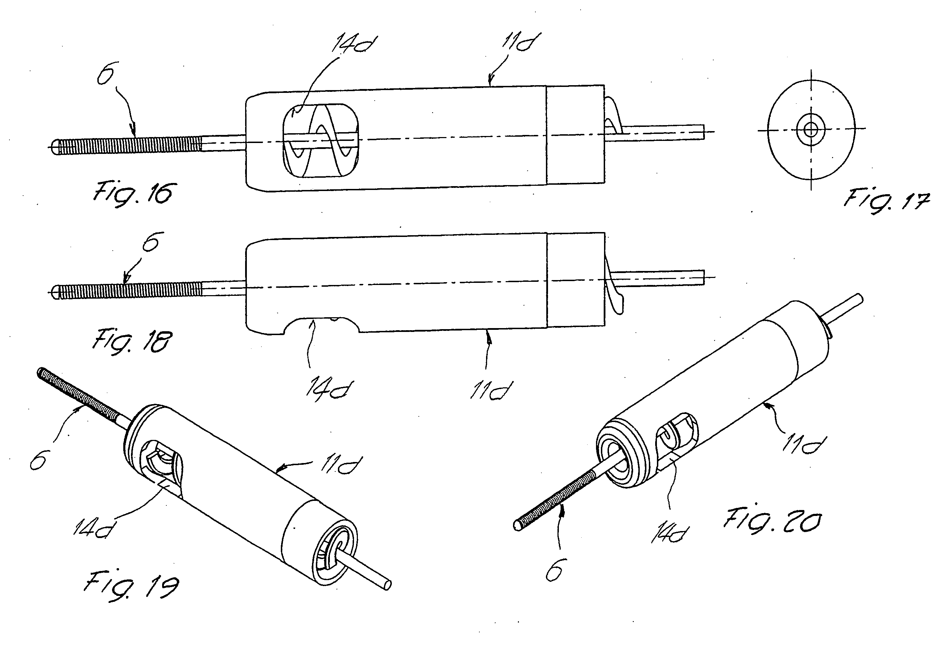

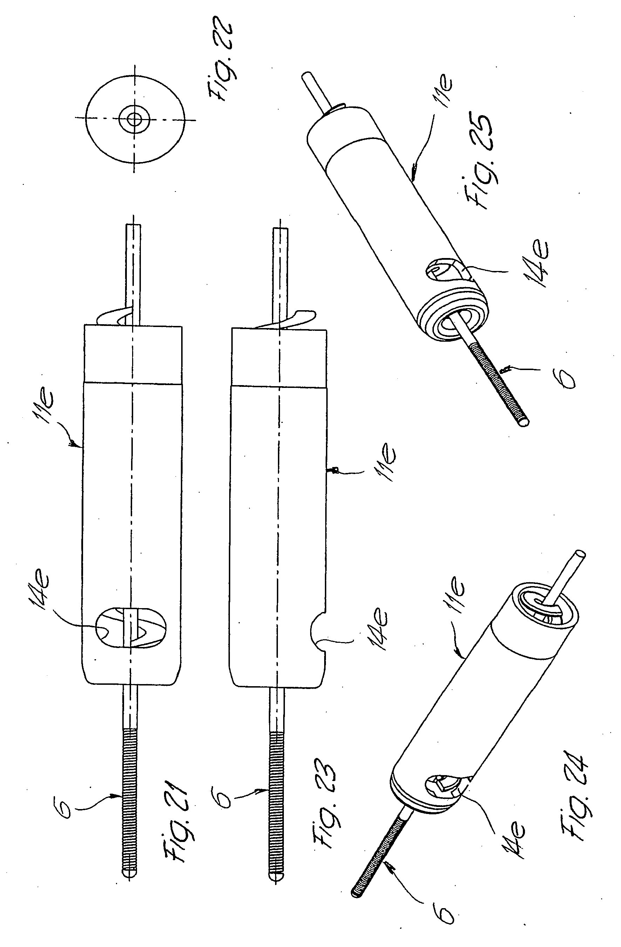

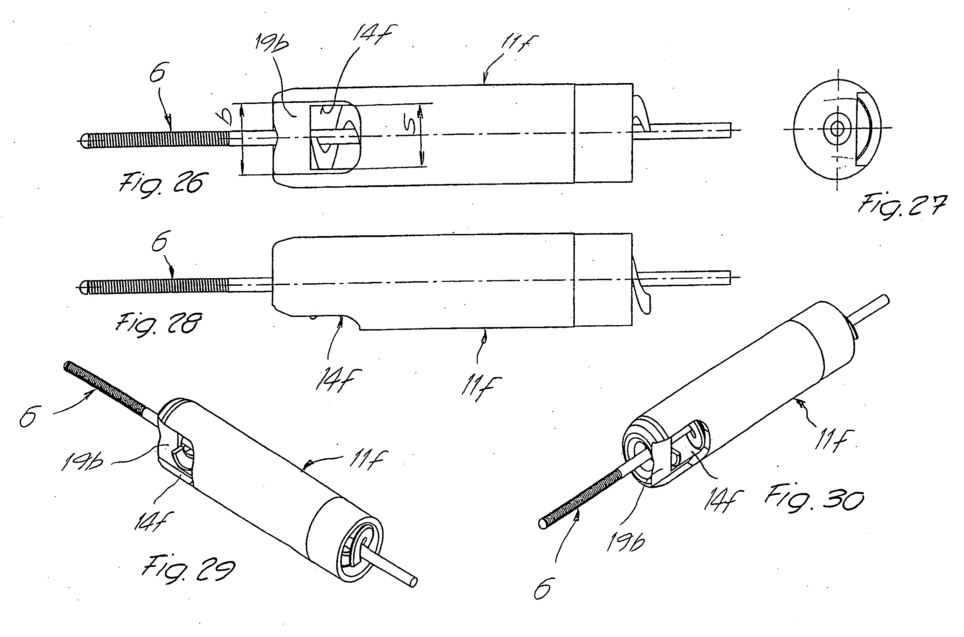

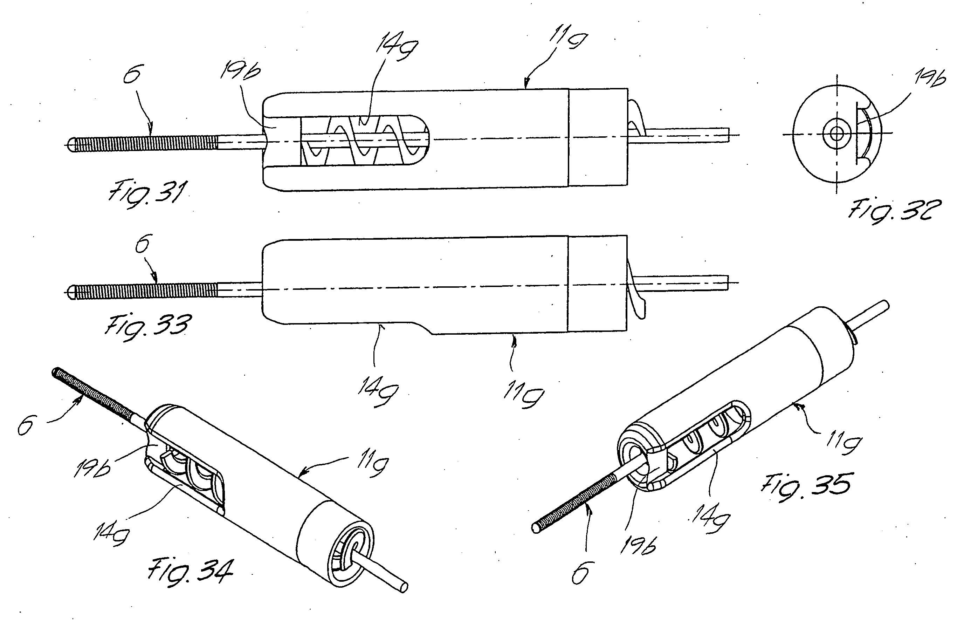

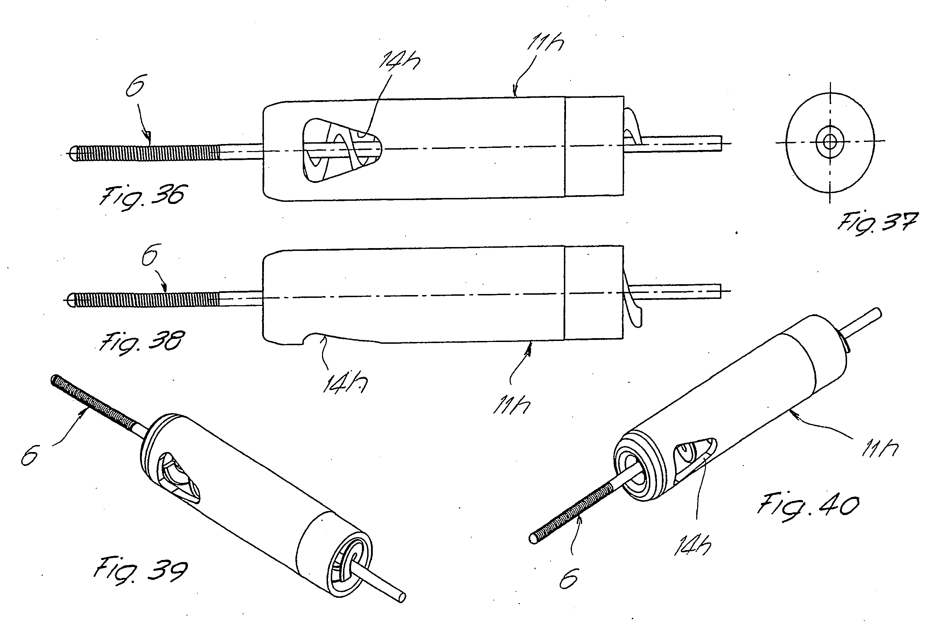

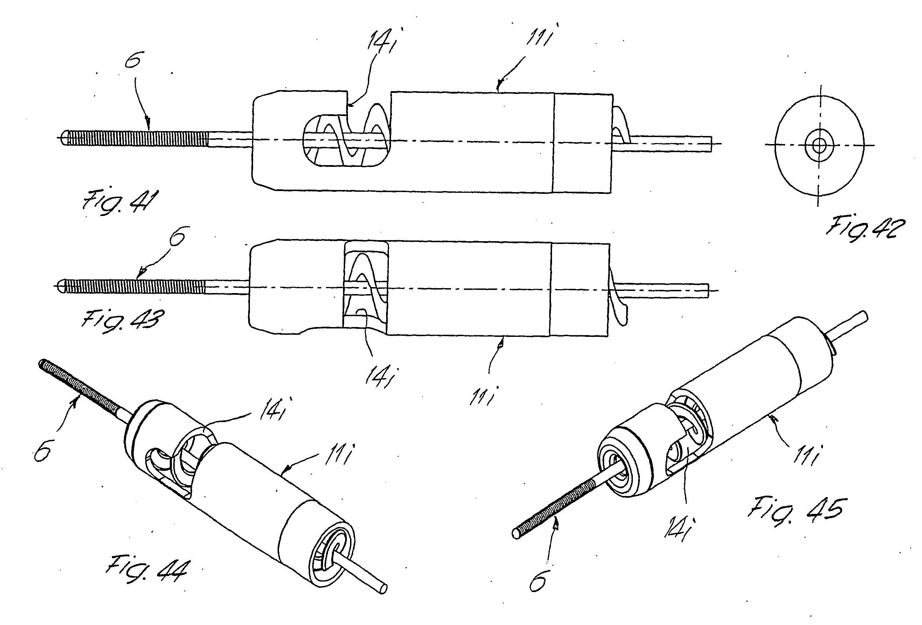

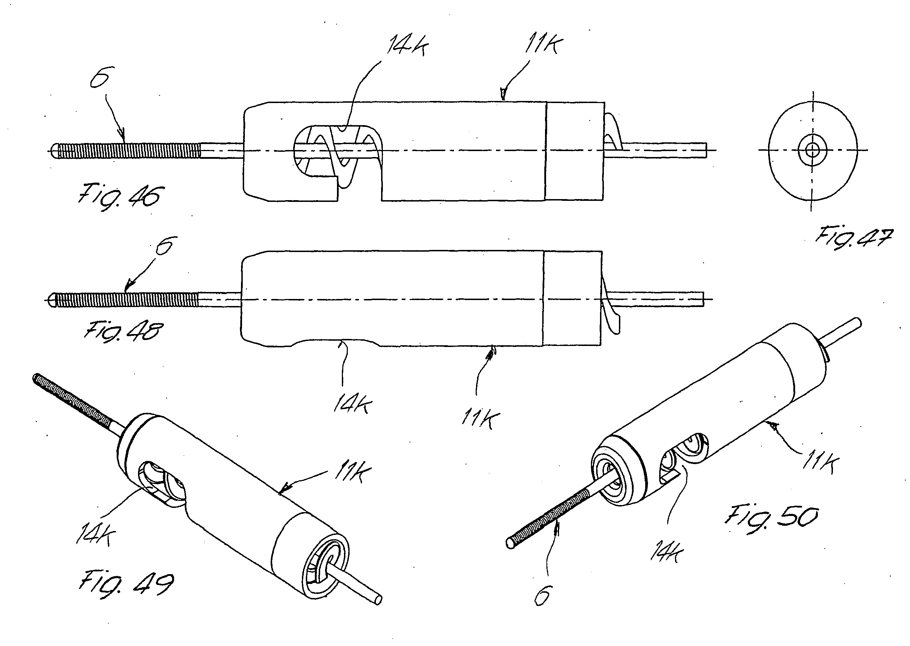

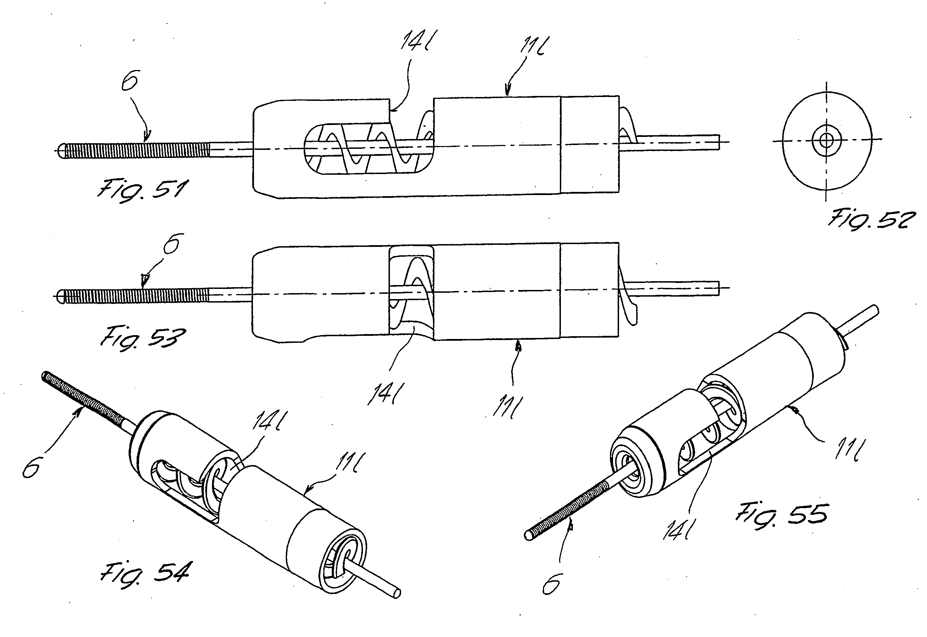

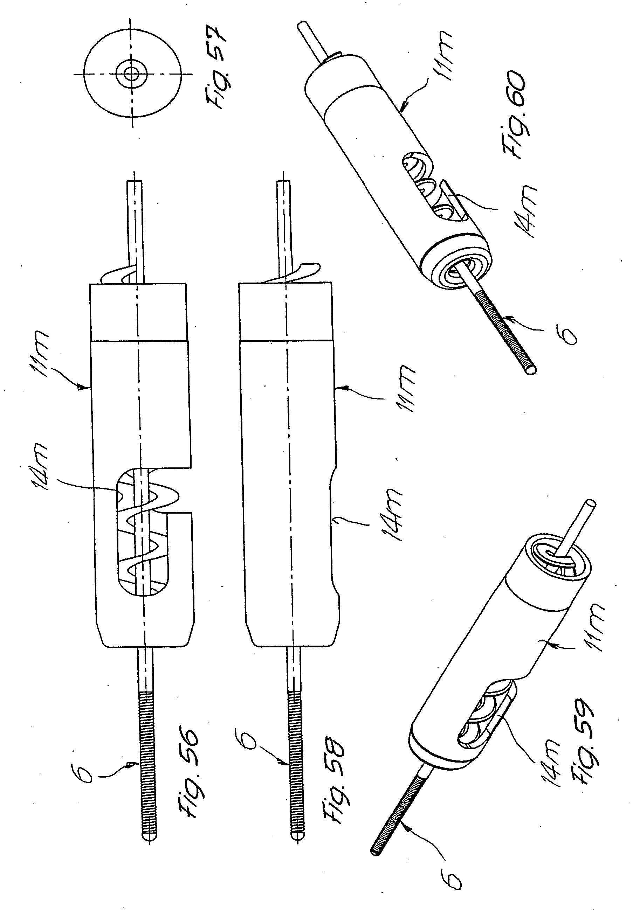

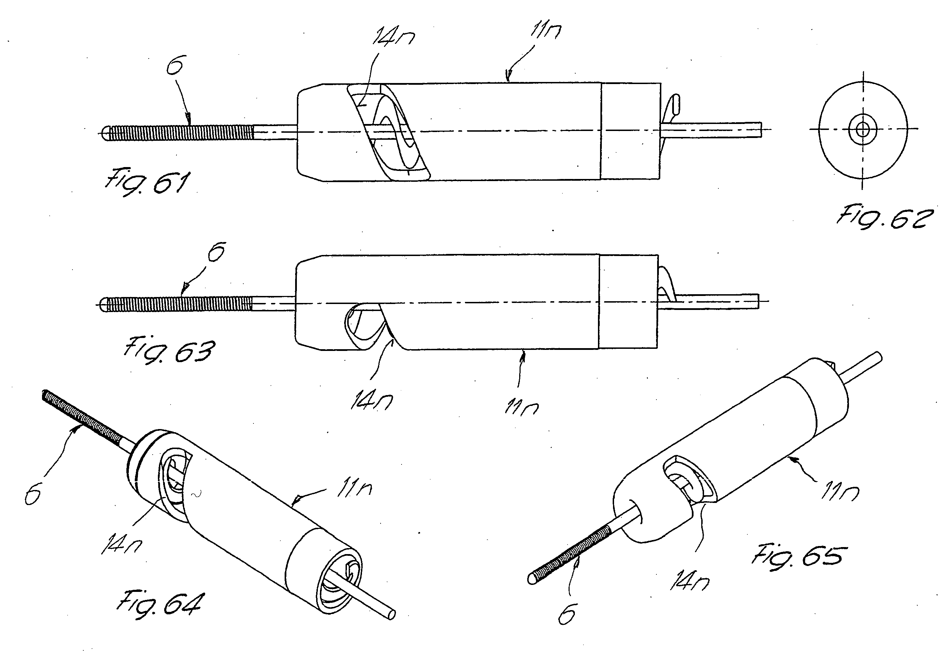

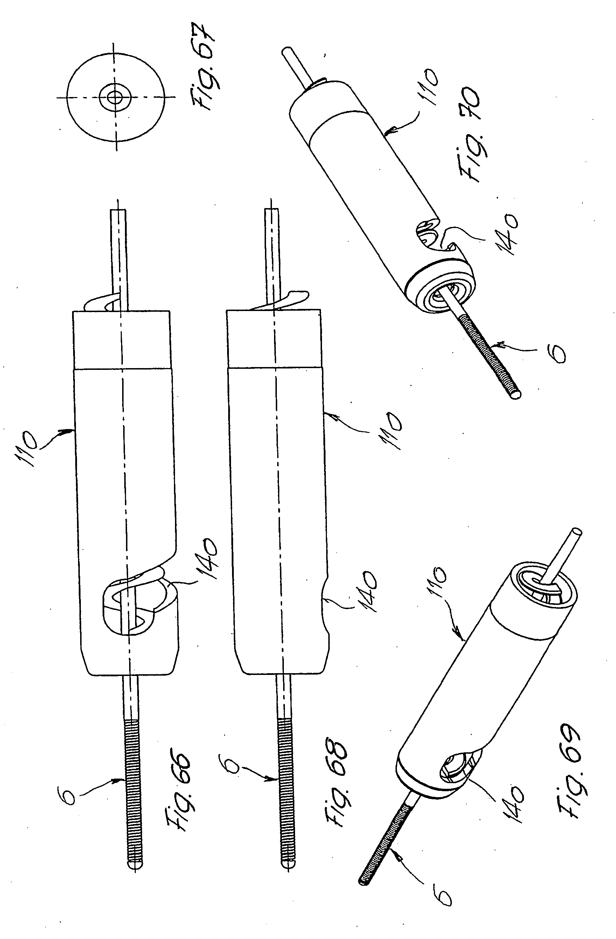

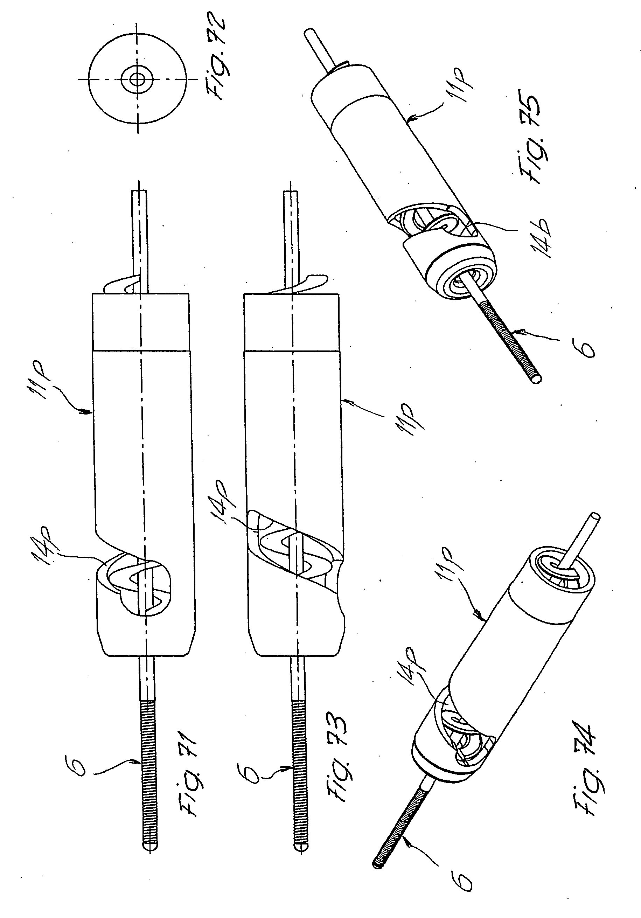

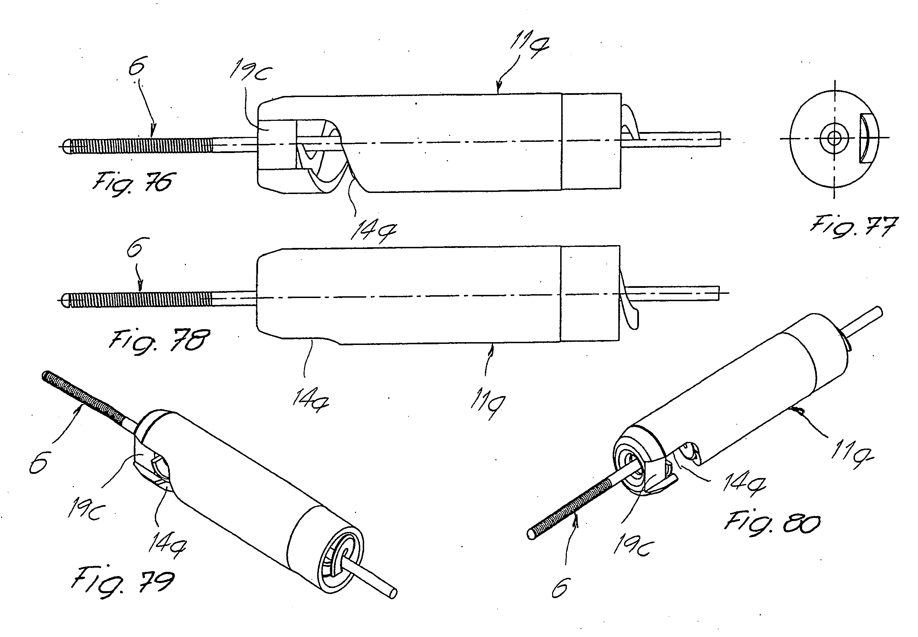

Die Fig. 6 bis 80 zeigen verschiedene Varianten für die Ausbildung der seitlichen Öffnung im Arbeitskopf. Diese Darstellungen sind jedoch nicht einschränkend, sondern nur beispielhaft zu verstehen. Weitere Ausführungsformen sowie auch Kombinationen der gezeigten Ausbildungen sind denkbar.

### Bezugszeichenliste

- 1: Antriebseinheit
- 2: Drehantrieb
- 3: Spritzkammer
- 4: Abfuhrkanal
- 5: Sammelbehälter
- 6: Führungsdraht
- 7: proximales Ende
- 8: distales Ende
- 9: Einführschleuse
- 10: Katheter
- 11a, 11b, 11c, 11d, 11e, 11f, 11g, 11h,:
- 11i, 11k, 11l, 11m, 11n, 11o, 11p, 11q: Arbeitskopf
- 12: Schlauch
- 13: Förderschraube
- 14a, 14b, 14c, 14d, 14e, 14f, 14g, 14h,:
- 14i, 14k, 14l, 14m, 14n, 14o, 14p, 14q: Öffnung
- 15: Kante
- 16: Rand
- 17: Armierung
- 18: Ummantelung
- 19a, 19b, 19c: nutförmige Ausnehmung
- 20: Längsschlitz
- 21: Umfangsschlitz

## Patentansprüche

1. Katheter zum Ansaugen, Fragmentieren und Hinausfördern von entfernbarem Material aus Hohlkörpern , insbesondere von Thromben und Embolien aus Blutgefässen, mit einem distalen und einem proximalen Ende, einem über einen Führungsdraht (6) unabhängig von diesem axial verschiebbaren, am distalen Ende des Katheters angeordneten Arbeitskopf (11), welcher wenigstens eine seitliche Öffnung (14) aufweist, wobei der Katheter (10) eine mittels eines vom Arbeitskopf (11) entfernten Drehantriebes (2) einer Antriebseinheit (1) in Rotation versetzbare flexible, sich vom proximalen zum distalen Ende des Katheters erstreckende Förderschraube (13) aufweist, wobei die Förderschraube (13) mit Förderflächen versehen ist, welche sich wendelförmig entlang ihrer Längsachse in der Richtung von Radien erstrecken, sowie mit einem die Förderschraube (13) umgebenden, mit dem Arbeitskopf (11) verbundenen flexiblen Schlauch (12) zur Abfuhr des Materials, bzw. der abgelösten Thromben und Emboliefragmente und einem Schneidwerkzeug und wobei die Förderschraube (13) im Bereich des Arbeitskopfes (11) als mit der Öffnung (14) des Arbeitskopfes (11) zusammenwirkendes, scherendes Schneidwerkzeug ausgebildet ist, das im Betriebszustand zwischen den peripheren Rändern (13a) der Förderschraube (13) und Rändern der Öffnungen (14) die eindringenden Materialien, bzw. angesaugten und/oder abgelösten Thromben und Embolien kontinuierlich zerkleinert und entlang der Förderfläche in Richtung des proximalen Endes (7) abführt, **dadurch gekennzeichnet, dass** die seitliche Öffnung (14) des Arbeitskopfes (11) als L-förmiger Schlitz (14i, 14k, 141, 14m) mit einem sich im wesentlichen in Längsrichtung und einem sich entlang eines Teils des Umfanges erstreckenden Schenkel ausgebildet ist..

2. Katheter nach Anspruch 1, **dadurch gekennzeichnet, dass** das Verhältnis der Breite des sich in Längsrichtung erstreckenden Schenkels zur Breite des sich in Umfangsrichtung erstreckenden Schenkels zwischen 1,0 und 1,3 beträgt.

3. Katheter nach Anspruch 1 oder 2, **dadurch gekennzeichnet, dass** der distale Teil der Förderschraube (13) im Bereich des Arbeitskopfes (11) im Aussendurchmesser gegenüber dem Innendurchmesser des vorzugsweise zylindrischen Arbeitskopfes (11) passgenau ausgebildet ist, so dass der Aussendurchmesser der Förderschraube (13) zum Innendurchmesser der Innenmantelfläche des Arbeitskopfes (11) ein nur minimales Durchmesserspiel aufweist.

4. Katheter nach einem der vorhergehenden Ansprüche, **dadurch gekennzeichnet, dass** die Kanten an der Aussenseite der Förderschraube (13) im Bereich der Öffnung (14) des Arbeitskopfes (11) scharfkantig ausgebildet sind.

5. Katheter nach einem der vorhergehenden Ansprüche, **dadurch gekennzeichnet, dass** der Arbeitskopf (11) gegen sein distales Ende (8) verjüngt ist

6. Katheter nach einem der vorhergehenden Ansprüche, **dadurch gekennzeichnet, dass** die Kanten (15) der seitlichen Öffnung (14a) im Bereich der Innenmantelfläche des Arbeitskopfes (11) wenigstens bereichsweise scharfkantig ausgebildet sind.

7. Katheter nach einem der vorhergehenden Ansprüche, **dadurch gekennzeichnet, dass** die Kanten (16) der seitlichen Öffnung (14a) im Bereich der Aussenmantelfläche des Arbeitskopfes (11a) wenigstens bereichsweise verrundet ausgebildet sind.

8. Katheter nach einem der vorhergehenden Ansprüche, **dadurch gekennzeichnet, dass** die seitliche Öffnung (14) als Schlitz ausgebildet ist

9. Katheter nach Anspruch 8, **dadurch gekennzeichnet, dass** der Schlitz wenigstens teilweise in axialer Richtung des Arbeitskopfes (11) verläuft.

10. Katheter nach Anspruch 8 oder 9, **dadurch gekennzeichnet, dass** der Schlitz (14n, 14o, 14p, 14q) relativ zur Längsachse des Arbeitskopfes (11 n, 11o, 11p, 11q) wenigstens zum Teil entlang einer Schraubenlinie ausgebildet ist

11. Katheter nach einem der vorhergehenden Ansprüche, **dadurch gekennzeichnet, dass** die Breite des Schlitzes (14h) zum proximalen Ende des Arbeitskopfes (11 h) hin abnimmt

12. Katheter nach einem der vorhergehenden Ansprüche, **dadurch gekenzeichnet, dass** im distalen Endbereich des Arbeitskopfes (11b, 11f, 11g, 11q) an der Aussenmantelfläche wenigstens eine vom distalen Ende ausgehende, in die seitliche Öffnung (14b, 14f, 14g, 14q) mündende nutförmige Ausnehmung (19a, 19b, 19c) angeordnet ist.

13. Katheter nach Anspruch 12, **dadurch gekennzeichnet, dass** die Tiefe der nutförmigen Ausnehmung (19) zum proximalen Ende des Arbeitskopfes hin zunimmt.

14. Katheter nach Anspruch 12 oder 13, **dadurch gekennzeichnet, dass** die Breite (b) der nutförmigen Ausnehmung (19b) grösser ist als die Sehne (s) des Innendurchmessers des Arbeitskopfes (11f) im Bereich des Nutbodens.

15. Katheter nach einem der vorhergehenden Ansprüche, **dadurch gekennzeichnet, dass** der Arbeitskopf (11a) mit dem Schlauch (12) axial zug- und druckfest verbunden ist

16. Katheter nach einem der vorhergehenden Ansprüche, **dadurch gekennzeichnet, dass** der Schlauch (12) wenigstens abschnittsweise eine Armierung (17) aufweist.

17. Katheter nach Anspruch 16, **dadurch gekennzeichnet, dass** die Armierung (17) als metallische Wendel ausgebildet ist.

18. Katheter nach Anspruch 16 oder 17, **dadurch gekennzeichnet, dass** die Armierung (17) auf der Innenseite des Schlauches (12) angeordnet ist

19. Katheter nach einem der vorgenannten Ansprüche, **dadurch gekennzeichnet, dass** der Schlauch (12) zweiteilig ausgebildet ist, wobei der proximale Teil als Kunststoffschlauch und der distale Teil als metallische Federwendel (17) mit dünnwandiger elastischer Kunststoff-Ummantelung (18) ausgebildet ist.

20. Katheter nach einem der vorgenannten Ansprüche, **dadurch gekennzeichnet, dass** der Arbeitskopf (11) und/oder die Förderschraube (13) aus Metall, insbesondere aus rostfreiem Stahl, bestehen.

21. Katheter nach einem der vorgenannten Ansprüche, **dadurch gekennzeichnet, dass** der Arbeitskopf (11) aus gesinterter Keramik oder Metallkeramik aufgebaut ist oder eine hochfeste Verscheiss-Schutzschicht aufweist.

## Claims

1. Catheter for aspirating, fragmenting and removing removable material from hollow bodies, in particular thrombi and emboli from blood vessels, with a distal end and a proximal end, a working head (11) which is arranged on the distal end of the catheter and is axially displaceable along a guide wire (6), independently thereof, and has at least one lateral opening (14), the catheter (10) having a flexible feed screw (13) which extends from the proximal end to the distal end of the catheter and which can be moved in rotation by means of a rotary drive (2) of a drive unit (1) remote from the working head (11), the feed screw (13) being provided with feed surfaces which extend helically along their longitudinal axis in the direction of radii, and with a flexible hose (12) which surrounds the feed screw (13), is connected to the working head (11) and is designed to remove the material or the detached thrombi and emboli fragments, and with a cutting tool, the feed screw (13) being designed in the area of the working head (11) as a shearing cutting tool which interacts with the opening (14) of the working head (11) and is designed, in the operating state, to continuously comminute materials or aspirated and/or detached thrombi and emboli penetrating between the peripheral edges (13a) of the feed screw (13) and the edges of the openings (14) and to convey them along the feed surface in the direction of the proximal end (7), **characterized in the** the lateral opening (14) of the working head (11) is designed as an L-shaped slit (14i, 14k, 14l, 14m) with a branch expending substantially in the longitudinal direction and with a branch expending about part of the circumference.

2. Catheter according to Claim 1, **characterized in that** the ratio of the width of the longitudinally expending branch to the width of the circumferentially expending branch is between 1.0 and 1.3.

3. Catheter according to Claim 1 or 2, **characterized in that** the distal part of the feed screw (13) in the area of the working head (11) has an external diameter that exactly fits the internal diameter of the preferably cylindrical working head (11), such that the external diameter of the feed screw (13) has only a minimal diameter play relative to the infernal diameter of the inner circumferential surface of the working head (11).

4. Catheter according to one of the preceding claims, **characterized in that** the edges on the outside of the feed screw (13) are formed so as to be sharp in the area of the opening (14) of the working head (11).

5. Catheter according to one of the preceding claims, **characterized in that** the working head (11) tapers towards its distal end (8).

6. Catheter according to one of the preceding claims, **characterized in that** the edges (15) of the lateral opening (14a), in the area of the inner circumferential surface of the working head (11), are formed so as to be sharp at least in sections.

7. Catheter according to one of the preceding claims, **characterized in that** the edges (16) of the lateral opening (14a), in the area of the outer circumferential surface of the working head (11a), are formed so as to be rounded at least in sections.

8. Catheter according to one of the preceding claims, **characterized in that** the lateral opening (14) is designed as a slit.

9. Catheter according to Claim 8, **characterized in that** the slit runs at least partially in an axial direction of the working head (11).

10. Catheter according to Claim 8 or 9, **characterized in that** the slit (14n, 14o, 14p, 14q) is formed, relative to the longitudinal axis of the working head (11n, 11o, 11p, 11q), at least partially along a helix.

11. Catheter according to one of the preceding claims, **characterized in that** the width of the slit (14h) decreases towards the proximal end of the working head (11h).

12. Catheter according to one of the preceding claims, **characterized in that** at least one groove-like recess (19a, 19b, 19c), which starts from the distal end and opens into the lateral opening (14b, 14f, 14g, 14q), is arranged in the distal end area of the working head (11b, 11f, 11g, 11q) on the outer circumferential surface.

13. Catheter according to Claim 12, **characterized in that** the depth of the groove-like recess (19) increases towards the proximal end of the working head.

14. Catheter according to Claim 12 or 13, **characterized in that** the width (b) of the groove-like recess (19b) is greater than the chord (s) of the internal diameter of the working head (11f) in the area of the groove base.

15. Catheter according to one of the preceding claims, **characterized in that** the working head (11a) is connected to the hose (12) axially in a manner resistant to tension and pressure.

16. Catheter according to one of the preceding claims, **characterized in that** the hose (12) has a reinforcement (17) at least in sections.

17. Catheter according to Claim 16, **characterized in that** the reinforcement (17) is designed as a metal coil.

18. Catheter according to Claim 16 or 17, **characterized in that** the reinforcement (17) is arranged on the inside of the hose (12).

19. Catheter according to one of the preceding claims, **characterized in that** the hose (12) is in two parts, of which the proximal part is designed as a plastic hose and the distal part is designed as a metal helical spring (17) having a thin elastic plastic sheath (18).

20. Catheter according to one of the aforementioned claims, **characterized in that** the working head (11) and/or the feed screw (13) are made of metal, in particular of stainless steel.

21. Catheter according to one of the aforementioned claims, **characterized in that** the working head (11) is made of sintered ceramic or metal ceramic or has a highly resistant layer for protection from wear.

## Revendications

1. Cathéter pour aspirer, fragmenter et évacuer de la matière extractible de corps creux, en particulier de thrombus et d'embolies dans des vaisseaux sanguins, comprenant une extrémité distale et une extrémité proximale, une tête de travail (11) déplaçable axialement par-dessus un câble de guidage (6) et indépendamment de celui-ci, disposée à l'extrémité distale du cathéter, laquelle présente au moins une ouverture latérale (14), le cathéter (10) présentant une vis de transport (13) flexible, déplaçable en rotation au moyen d'un entraînement en rotation (2) d'une unité d'entraînement (1), éloigné de la tête de travail (11), s'étendant depuis l'extrémité proximale jusqu'à l'extrémité distale du cathéter, la vis de transport (13) étant pourvue de surfaces de transport qui s'étendent en hélice le long de leur axe longitudinal dans la direction de rayons, ainsi que d'un tuyau flexible (12) entourant la vis de transport (13), connecté à la tête de travail (11) pour l'évacuation de la matière, ou des thrombus et fragments d'embolie décollées, et d'un outil de coupe, la vis de transport (13) étant réalisée dans la région de la tête de travail (11) sous forme d'outil de coupe cisaillant coopérant avec l'ouverture (14) de la tête de travail (11), lequel broie en continu, dans son état de fonctionnement entre les bords périphériques (13a) de la vis de transport (13) et les bords des couvertures (14) les matières entrantes, ou les thrombus et embolies aspirés et/ou décollés et les évacue le long de la surface de transport dans la direction de l'extrémité proximale (7), **caractérisé en ce que** l'ouverture latérale (14) de la tête de travail (11) est réalisée sous forme de fente en forme de L (14i, 14k, 141, 14m) avec une branche s'étendant essentiellement dans la direction longitudinale et une branche s'étendant le long d'une partie de la périphérie.

2. Cathéter selon la revendication 1, **caractérisé en ce que** le rapport de la largeur de la branche s'étendant dans la direction longitudinale à la largeur de la branche s'étendant dans la direction périphérique est compris entre 1,0 et 1,3.

3. Cathéter selon la revendication 1 ou 2, **caractérisé en ce que** la partie distale de la vis de transport (13) dans la région de la tête de travail (11) est réalisée en termes de diamètre extérieur avec un ajustement étroit au diamètre intérieur de la tête de travail (11) de préférence cylindrique, de sorte que le diamètre extérieur de la vis de transport (13) ne présente, par rapport au diamètre intérieur de la surface d'enveloppe intérieure de la tête de travail (11), qu'un jeu minimal entre les diamètres.

4. Cathéter selon l'une quelconque des revendications précédentes, **caractérisé en ce que** les arêtes au niveau du côté extérieur de la vis de transport (13) sont réalisées avec des arêtes vives dans la région de l'ouverture (14) de la tête de travail (11).

5. Cathéter selon l'une quelconque des revendications précédentes, **caractérisé en ce que** la tête de travail (11) est rétrécie vers son extrémité distale (8).

6. Cathéter selon l'une quelconque des revendications précédentes, **caractérisé en ce que** les arêtes (15) de l'ouverture latérale (14a) sont réalisées avec des arêtes vives au moins par zones dans la région de la surface d'enveloppe intérieure de la tête de travail (11).

7. Cathéter selon l'une quelconque des revendications précédentes, **caractérisé en ce que** les arêtes (16) de l'ouverture latérale (14a) sont réalisées sous forme arrondie au moins par zones dans la région de la surface d'enveloppe extérieure de la tête de travail (11a).

8. Cathéter selon l'une quelconque des revendications précédentes, **caractérisé en ce que** l'ouverture latérale (14) est réalisée sous forme de fente.

9. Cathéter selon la revendication 8, **caractérisé en ce que** la fente s'étend au moins en partie dans la direction axiale de la tête de travail (11).

10. Cathéter selon la revendication 8 ou 9, **caractérisé en ce que** la fente (14n, 14o, 14p, 14q) est réalisée au moins en partie le long d'une ligne hélicoïdale par rapport à l'axe longitudinal de la tête de travail (11n, 11o, 11p, 11q).

11. Cathéter selon l'une quelconque des revendications précédentes, **caractérisé en ce que** la largeur de la fente (14h) diminue vers l'extrémité proximale de la tête de travail (11h).

12. Cathéter selon l'une quelconque des revendications précédentes, **caractérisé en ce que** dans la région d'extrémité distale de la tête de travail (11b, 11f, 11g 11q) au niveau de la surface d'enveloppe extérieure, est disposé au moins un évidement (19a, 19b, 19c) en forme de rainure partant de l'extrémité distale et débouchant dans l'ouverture latérale (14b, 14f, 14g, 14q).

13. Cathéter selon la revendication 12, **caractérisé en ce que** la profondeur de l'évidement en forme de rainure (19) augmente vers l'extrémité proximale de la tête de travail.

14. Cathéter selon la revendication 12 ou 13, **caractérisé en ce que** la largeur (b) de l'évidement en forme de rainure (19b) est supérieure à la corde (s) du diamètre inférieur de la tête de travail (11f) dans la région du fond de rainure.

15. Cathéter selon l'une quelconque des revendications précédentes, **caractérisé en ce que** la tête de travail (11a) est connectée axialement de manière rigide en fraction et en compression au tuyau (12).

16. Cathéter selon l'une quelconque des revendications précédentes, **caractérisé en ce que** le tuyau (12) présente au moins en partie une armure (17).

17. Cathéter selon la revendication 16, **caractérisé en ce que** l'armure (17) est réalisée sous forme d'hélice métallique.

18. Cathéter selon la revendication 16 ou 17, **caractérisé en ce que** l'armure (17) est disposée du côté intérieur du tuyau (12).

19. Cathéter selon l'une quelconque des revendications précédentes, **caractérisé en ce que** le tuyau (12) est réalisé en deux parties, la partie proximale étant réalisée sous forme de tuyau en plastique et la partie distale sous forme d'hélice à ressort (17) avec une gaine en plastique élastique à parois minces (18).

20. Cathéter selon l'une quelconque des revendications précédentes, **caractérisé en ce que** la tête de travail (11) et/ou la vis de transport (13) se composent de métal, en particulier d'acier inoxydable.

21. Cathéter selon l'une quelconque des revendications précédentes, **caractérrisé en ce que** la tête de travail (11) est construite en céramique frittée ou en métal-céramique, ou présente une couche de protection contre l'usure à grande résistance.
